# EUROPEAN PATENT APPLICATION

(11) **EP 3 753 527 A1**
(43) Date of publication of application: **23.12.2020**
(21) Application number: 19382528.8
(22) Date of filing: 21.06.2019
(51) Int. Cl.: A61C 9/00

(54) **REMOVABLE FIXING DEVICE FOR EDENTULOUS CASES**

(71) Applicant: Cabaro Dental, SL, 35002 Las Palmas de Gran Canaria (ES)
(72) Inventor: Caballero de Rodas Ramírez, Alfonso, 35002 Las Palmas de Gran Canaria (ES)
(74) Representative: Carlos Hernando, Borja

(57) **Abstract**

The removable fixing device for edentulous cases, to be applied on the bone of a toothless gum, or on the depiction of a toothless gum, in the mandible and/or maxilla, relates to an element which is temporarily fixed to a depiction of a gum and/or to the bone of a gum of a patient. Said device is used in an image guided navigation system which enables tracking and depicting the movement of a tool during oral surgery to be carried out. Said fixing device presents two preferred shapes, a first shape, a master device, used for being fixed to a depiction of a gum and obtaining from same the second preferred shape, a surgical device, by means of adding material on the master device for later scanning and printing of the surgical device.

## Description

### OBJECT OF THE INVENTION

The present invention, a removable fixing device for edentulous cases, to be applied on the bone of a toothless gum, or on the depiction of a toothless gum, in the mandible and/or maxilla, relates to an element which is temporarily fixed to a depiction of a gum and/or to the bone of a gum of a patient. Said device is used in an image guided navigation system which enables tracking and depicting the movement of a tool during oral surgery to be carried out. Said fixing device presents two preferred shapes, a first shape, a master device, used for being fixed to a depiction of a gum and obtaining from same the second preferred shape, a surgical device, by means of adding material on the master device for later scanning and printing of the surgical device.

The invention is applicable in the sector of dental surgical equipment for image guided systems.

### BACKGROUND OF THE INVENTION

Different solutions for image guided surgery are known in the state of the art using a computerised axial tomography (CAT) scan to create a digital image of the surgical space. An example of solutions of this type is described in international application number WO2014152519A1.

Said document describes a fixing device for being used with an image guided navigation system for tracking and depicting the movement of a tool during oral surgery. In particular, said device is fixed to a tooth and then coupled to the fixing device, through a support, a tracking component of the guidance system. Said component preferably comprises an arm coupled to the support. A description of said fixing device on a tooth and of the navigation system can be found in said document WO2014152519A1.

The aforementioned fixing device offers good results when there is a tooth existing in the mandible or maxilla where the surgery is to be performed to which the fixing device can be fixed. However, in the case of edentulous, or toothless patients, the aforementioned fixing device is not viable in the mandible and/or maxilla because it cannot be fixed to the gum, or better yet to the bone of the gum, ensuring the position of the fixing device with respect to the oral cavity for the mentioned navigation system to subsequently be used.

The present invention proposes a removable fixing device for edentulous cases. Specifically, it proposes a first device or master device which is fixed to the depiction of a gum for obtaining a second device or surgical device which is fixed to the bone of a gum of a patient. The position of the markers for later use thereof in the image guided navigation system and the coupling thereto of a tracking component of the navigation system are ensured by means of this surgical fixing device.

### DESCRIPTION OF THE INVENTION

A first object of the present invention, a removable fixing device for edentulous cases, is a device according to claim 1.

The object of said first claim is a removable fixing device for edentulous cases for being used together with an image guided navigation system for tracking and depicting the movement of a tool during oral surgery. Specifically, the device can be used to be fixed to the bone of a toothless gum of a patient or to be fixed to a depiction of a toothless gum. Said device presents a body with at least:
- a substantially vertical wall for being opposite the substantially vertical surface of a gum or of the depiction of a gum,
- an upper surface extending from the rear part of the wall, forming an approximate right angle with said wall, to be arranged on the occlusal surface of the gum or of the depiction of the gum, and
- a support in the front part of the curved wall for holding a component of the image guided navigation system. Said component of the navigation system could be a tracking element, a camera or another element, and it preferably presents an arm for being coupled through same to the support of the fixing device. Likewise, said support could have different configurations depending on the coupling means used by the component of the navigation system.

The wall of the device can present side extensions such that the surface thereof is curved to be adapted to the substantially vertical surface of the gum or to the surface of the depiction of the gum.

The upper surface can either comprise or be formed by at least one arm extending from the rear part of the substantially vertical wall to a free end also placed in the rear part of said wall. Said arm will be placed in one position or in another along the wall of the device depending on in which site of the gum one or several implants will be introduced. Likewise, said arm could be straight, L-shaped or even T-shaped, among other possible shapes for the purpose of placing the markers which will serve to determine the spatial position of the oral fixing device in an image guided navigation system.

As mentioned, the fixing device preferably incorporates markers, preferably at least three, on the body of the device which are used to determine the spatial position or placement of the fixing device with respect to the mandible or maxilla of a patient, and specifically with respect to the bone of the gum where a fixing device is arranged on the gum of a patient. Said markers can be made of a material with a density different from that of the body of the device in order to thus be detected by the scanner. They will preferably be spherical radiological markers, preferably made of a metal material. The arrangement of the markers must always be the same, depending on the image guided navigation system, in each fixing device to enable interaction with the navigation system, such that the markers are preferably arranged on the upper surface, two of them on the upper surface close to the wall and the third one at the end of the upper surface made up of the arm, the distances between said markers always being the same. Preferably the markers are spheres, though they could have other shapes.

Alternatively, it is possible to have a fixing device without markers on which the markers will later be arranged, as if it were an intermediate product. To that end, said fixing device will comprise in its body three housings, the geometry of which corresponds with that of the markers which will later be included in said housings, such that if the markers are spherical, the housings will present a shape of a semispherical cavity. In the event that housings are arranged only for the markers, the position thereof will be the same that the markers must have once they are installed, that is, they must be placed on the body of the device with the same separation, and preferably two housings on the upper surface arranged on the wall or close to the wall and a third housing at the free end of the arm. The markers can then be arranged in the housings of that device for use during oral surgery of a patient.

In this manner and according to the foregoing, there are preferably two types of fixing devices, a first fixing device or master fixing device, the purpose of which is to determine a shape and geometry of the prior initial device for the later manufacture of a second device or surgical fixing device.

The master fixing device comprises a substantially vertical wall with a horizontal surface forming an approximate right angle with said wall, said approximate right angle being between 80° and 100°. Said angle depends on the angle existing between the vertical wall and the occlusal surface of the gum of the patient. Said master device, preferably manufactured from an acrylic resin or like material, is used to be arranged on a three-dimensional depiction of the gum of the patient for the purpose of designing on the depiction of the gum the specific shape of the surgical fixing device which will be adapted to the surgery to be performed on the patient. The master device is thereby fixed to the wall of the depiction of the gum by means of an acrylic resin or like material. Alternatively other fixing means can be used. Said master fixing device will incorporate at least one arm on the upper surface thereof for the arrangement of a marker or a housing for a marker. The placement of the arm will depend on the part of the occlusal surface of the gum in which the implant will be provided by placing the arm having a specific shape depending on the location of the device in the gum and on the placement of the implant in the gum. The master device may or may not incorporate the positioning markers, though it must at least incorporate the housings for said markers.

Based on said master fixing device, and after it is fixed to the depiction of the gum of the patient through suitable retaining means, material, preferably an acrylic resin or like material, is added to same to adapt the shape of said master device to the specific shape of the gum. Material will primarily be added on both sides of the wall such that a curved wall adapted to the shape of the gum is formed, increasing the contact surface between the fixing device and the depiction of the gum. Material could also be added to the upper surface of the device if considered necessary. Thereby, the rear part of the wall reflects the imprint of the vestibular part of the gum of the patient, with each surgical fixing device thus being customised to each patient. Based on this variation of the master device, said device will be scanned to later print, preferably in medical-surgical grade resin or the like in three dimensions, the surgical oral fixing device which will be used on the gum of the patient and will be fixed to the bone of said gum. Once the body of the surgical device has been obtained, the location markers will be arranged on the housings thereof, preferably by means of a suitable adhesive, to complete said surgical fixing device. Said surgical oral fixing device will be fixed to the bone of the gum preferably by means of screwing which will go through the wall of the device until being screwed to the bone of the patient. As many screws as are needed to ensure the position of the fixing device in the bone of the gum could be used. Other alternative fixing systems to screwing could be used provided that the position of the fixing device in the bone is ensured since it will be the reference of the image guided navigation system in which said device will be used.

In any event, and depending on the placement, a master fixing device could sometimes be used as a surgical fixing device, primarily in those cases in which it is not necessary to increase the contact surface of the wall of the device with the gum, such that the master fixing device could also be the surgical fixing device and be fixed not only to the depiction of the gum but also to the bone of the gum of the patient. To use the master device as a surgical device the markers will need to be incorporated in the housings of the device.

It must also be taken into account that on said fixing device, both on the master device and on the surgical device, there can be coupled, specifically on the support arranged in the wall of the device, a tracking component of the image guided navigation system which may affect the stresses and the positioning of the device on the depiction of the gum and on the bone of the gum itself.

After performing surgery, the surgical retaining device is removed or separated from the bone of the gum.

Moreover, a second object of the invention is a method for manufacturing a fixing device according to claim 12. According to the foregoing, the method for manufacturing an oral fixing device for an image guided navigation system would comprise the following steps:
a) Taking a digital impression of the toothless gum of a patient,
b) Printing the depiction of the gum of the patient in a suitable material,
c) Arranging through retaining means a fixing device according to at least claim 1 on the depiction of the printed gum,
d) Adding material to the wall of the fixing device to adjust the shape of said wall to the curved shape of the substantially vertical wall of the depiction of the gum and to the surgery to be performed,
e) Solidifying/ curing the material added to the fixing device,
f) Separating the fixing device from the depiction of the gum,
g) Scanning said fixing device,
h) Printing the fixing device to obtain a second fixing device according to at least claim 2.

It is possible that immediately before or immediately after step c) a tracking component of the image guided navigation system is coupled to the support arranged in the front part of the wall of the device to ensure the correct design and positioning of the master fixing device in the depiction of the gum and then of the surgical fixing device in the bone of the gum.

Alternatively, it is possible for steps a) to g) of the above method to be performed digitally, that is, based on a first digitalised oral fixing device, or digitalised master device, and the digitalised gum, together with suitable computerised means, said first digitalised master device is adapted to the gum for digitally designing a surgical fixing device later printed in 3D for use on the gum of the patient.

Likewise, is possible after fixing the surgical fixing device on the gum, to scan the entire assembly of the gum and fixing device again and merge the computerised axial tomography (CAT) scan of the bone of the patient with the assembly of the gum and fixing device; and thus digitally plan the surgery. Therefore, by computer means, a computer and suitable software, the sites where the implants will be placed in the gum are marked by means of posts. The image guided navigation system serving as guidance in surgery will thereby know in which sites the implants are to be provided after fixing the surgical device to the bone of the gum again. This option is also possible in the case described in detail in the preceding paragraph, that is, once the digital surgical fixing device is available, the surgery is planned digitally and prior to the 3D printing of the surgical fixing device.

Then once the second fixing device is manufactured, the positioning or locating markers could be added on the housings for use in oral surgery by means of an image guided navigation system.

### DESCRIPTION OF THE FIGURES

To complement the description being made and for the purpose of helping to better understand the features of the invention, a set of drawings is included in which the following is depicted in an illustrative and non-limiting manner:
Figure 1 shows a perspective view of a master device according to the present invention.
Figure 2 shows a rear perspective view of the device of Figure 1.
Figure 3 shows a perspective view of an alternative master device.
Figure 4 shows a rear perspective view of the device of Figure 3.
Figure 5 shows a perspective view of a surgical device according to the present invention.
Figure 6 shows a rear perspective view of the device of Figure 5.

### PREFERRED EMBODIMENT OF THE INVENTION

Different embodiments of the objects of the present invention will be described below in reference to the aforementioned figures.

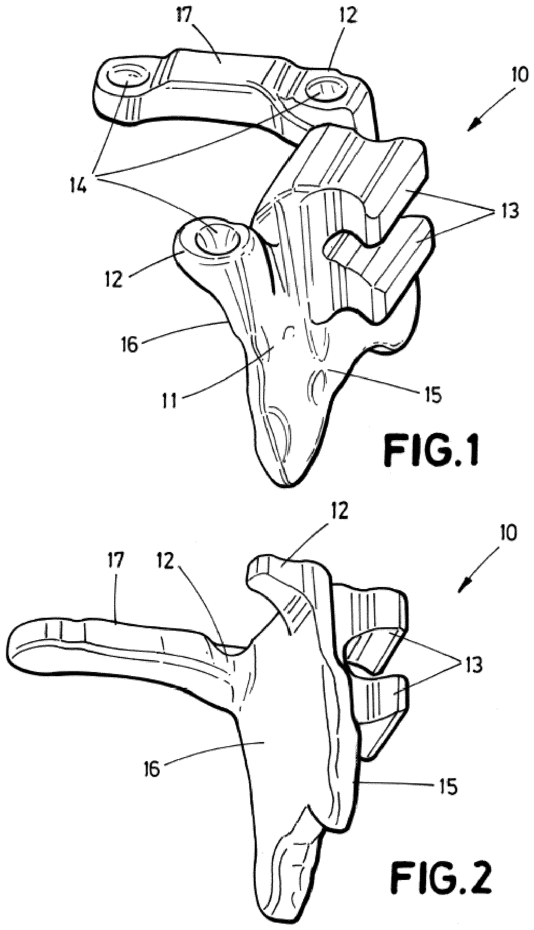

Figures 1 and 2 show a first example of a first fixing device or master fixing device 10, prior to arrangement thereof on a depiction of a gum. In said figures a body 10 with a substantially vertical wall 11, with a front part 15 and a rear part 16, and with an upper surface 12 forming an angle of between 80° and 100° with said substantially vertical wall 11 can be observed. There is arranged in the front part 15 of said wall a support 13 made up of, for example, a projection with a guide for the coupling of a component of the image guided navigation system in which the fixing device will be used. The configuration of said support 13 may vary depending on the coupling means arranged on the component.

The upper surface 12 presents a straight arm 17 placed at one end of the wall 11 and extending from the rear part 16 of said wall 11 for placing at its free end a housing 14 for a positioning marker, which will preferably be a spherical radiological marker, preferably made of a metal material (not shown). Other housings 14 for positioning markers are placed on the upper surface 12 in the part close to the wall 11. The housings 14 for said markers are semispherical, so the markers will preferably be spherical to be adapted to said housings. Other housing and marker shapes are possible.

The wall 11 of said device 10 is fixed to the vertical wall of the depiction of the gum by means of an acrylic resin.

The fixing device 10 could, in addition to being used as a master device, be used as a surgical device, that is, it could be fixed directly to the bone of the gum, depending on the case.

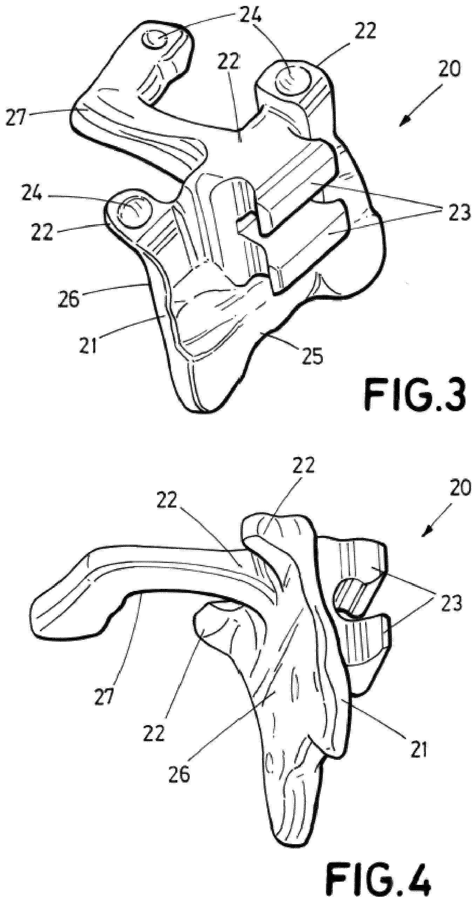

Figures 3 and 4 show a second embodiment of an oral fixing master device 20, similar to that of Figures 1 and 2, but comprising an upper surface 22 with an L-shaped arm 27. Likewise, said arm 27 extends from about the halfway point of the rear part 26 of the wall 21. The rest of the elements of this second example of a master device 20 are similar to the master device of Figures 1 and 2, that is, a body 20 with a substantially vertical wall 21 and an upper surface 22 forming an approximate right angle, between 80° and 100°, with the wall 21. Said wall 21 presents a rear part 26 and a front part 25 with a support 23 for a component of an image guided navigation system. The configuration of said support 13 may vary depending on the coupling means arranged on the component. Likewise, there are arranged on the upper surface 22, including the arm 27, housings 24 for the positioning markers (not shown). The shape of said housings 24 will depend on the shape of the markers. The wall 21 is fixed to the wall of the depiction of the gum by means of an acrylic resin.

Like the fixing device 10 of the first example, the fixing device 20 could, in addition to being used as a master device, be used as a surgical device, that is, it could be fixed directly to the bone of the gum depending on the case, and provided that a suitable material, preferably medical-surgical grade resin, is used in the manufacture thereof.

The preceding examples show two arrangements and possible shapes of two arms 17, 27, although other configurations would be possible depending on the surgical needs of each case.

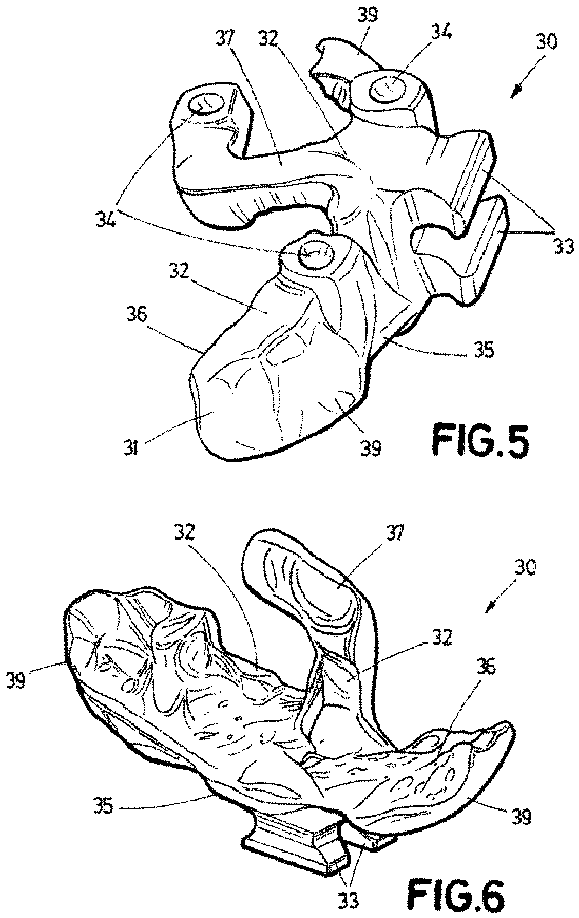

Figures 5 and 6 show an embodiment of a second holding device or surgical oral holding device 30 object of the invention. Said device presents a body 30 with a substantially vertical wall 31 which presents curved side extensions 39 to be adapted to the shape of the gum of the patient. Said wall 31 presents a rear part 36 and a front part 35 in which there is arranged a support 33 for the coupling of a component of the image guided navigation system. Said rear part 36 of the wall 31 reflects the imprint of the vestibular part of the gum of the patient, each surgical fixing device thus being customised to each patient. Additionally, and forming an approximate right angle, between 80° and 100°, with said wall, the body 30 presents an upper surface 32 extending from the rear part 36 of the wall 31. Said upper surface 32 presents an L-shaped arm 37 arranged in approximately the central part of the wall 31. The arm could be placed in other sites of the wall 31 and it could present another configuration, for example, it could be straight or T-shaped, depending on the surgical needs of the patient.

There are arranged on said upper surface 32 and on the free end of the arm 37 housings 34 for the positioning markers (not shown), the shape of which in this case is semispherical for receiving spherical markers, although said shape may vary depending on the shape of the marker.

## Claims

1. A removable fixing device for edentulous cases, to be fixed to the bone of a toothless gum of a patient or to be fixed to a depiction of a toothless gum, of an image guided navigation system for tracking and depicting the movement of a tool during oral surgery, **characterised in that** it comprises a body with:
- a wall,
- an upper surface extending from the rear part of the wall, forming an approximate right angle with said wall, to be arranged on the occlusal surface of the gum or of the depiction of the gum, and
- a support in the front part of the curved wall for holding a component of the image guided navigation system.

2. The device according to claim 1, **characterised in that** the wall presents a curved surface, the curvature of the wall being adapted to the substantially vertical surface of the gum or of the depiction of the gum.

3. The device according to any of the preceding claims, **characterised in that** the upper surface comprises an arm extending from the rear part of the wall to a free end also placed in the rear part of said wall.

4. The device according to any of the preceding claims, **characterised in that** the arm is straight, L-shaped or T-shaped.

5. The device according to any of the preceding claims, **characterised in that** the body comprises at least three markers to determine the spatial placement of the fixing device with respect to the bone of the gum.

6. The device according to claim 5, **characterised in that** it comprises a marker placed at the free end of the horizontal arm and two markers in the horizontal surface.

7. The device according to any of claims 1 to 4, **characterised in that** the body comprises at least three separated housings of markers to determine the placement of the fixing device with respect to the bone of the gum.

8. The device according to claim 7, **characterised in that** it comprises a housing placed at the free end of the horizontal arm and two housings in the horizontal surface.

9. The device according to claim 8, **characterised in that** the housings comprise markers.

10. The device according to any of the preceding claims, **characterised in that** the material is medical-surgical grade resin.

11. The device according to any of the preceding claims, **characterised in that** the approximate right angle between the upper surface and the wall is between 80° and 100°.

12. A method for manufacturing an oral fixing device for edentulous cases of an image guided navigation system for tracking and depicting the movement of a tool during oral surgery, **characterised in that** it comprises the following steps:
a) Taking a digital impression of the toothless gum of a patient,
b) Printing the depiction of the gum of the patient in a suitable material,
c) Arranging through retaining means a fixing device according to at least claim 1 on the depiction of the printed gum,
d) Adding material to the wall of the fixing device to adjust the shape of said wall to the curved shape of the substantially vertical wall of the depiction of the gum and to the surgery to be performed,
e) Solidifying/ curing the material added to the fixing device,
f) Separating the fixing device from the depiction of the gum,
g) Scanning said fixing device,
h) Printing the fixing device to obtain a second fixing device according to at least claim 2.
